# EUROPEAN PATENT SPECIFICATION

(11) **EP 3 708 200 B1**
(45) Date of publication and mention of the grant of the patent: **01.05.2024**
(21) Application number: 19163052.4
(22) Date of filing: 15.03.2019
(51) Int. Cl.: A61M 5/00, A61M 5/32

(54) **PACKAGE FOR A PREFILLED SYRINGE AND SYSTEM COMPRISING A PREFILLED SYRINGE AND A PACKAGE**
VERPACKUNG FÜR EINE VORGEFÜLLTE SPRITZE UND SYSTEM MIT EINER VORGEFÜLLTEN SPRITZE UND EINER VERPACKUNG
EMBALLAGE POUR SERINGUE PRÉREMPLIE ET SYSTÈME COMPRENANT UNE SERINGUE PRÉREMPLIE ET UN EMBALLAGE

(43) Date of publication of application: 16.09.2020
(73) Proprietor: Gerresheimer Regensburg GmbH, 93047 Regensburg (DE)
(72) Inventor: Pfrang, Jürgen, 93183 Kallmünz (DE); Gorshöfer, Andreas, 93149 Nittenau (DE)
(74) Representative: Hannke Bittner & Partner mbB Regensburg

(56) References cited:
- WO-A1-2014/045336
- US-A1- 2004 238 391
- US-A1- 2014 224 688
- US-A1- 2016 375 199
- US-A1- 2017 143 893
- US-A1- 2017 367 780

## Description

The invention relates to a system comprising a prefilled syringe with a syringe barrel and a syringe tip and a package suitable and intended for receiving the prefilled syringe, wherein the syringe tip is provided with a tip cap.

Prefilled syringes are delivered to the user filled with the medium to be administered. Nowadays, such syringes have emerged as one of the fastest-growing choices for unit dose medication. The handling of such syringes is easy because the medium to administer does not need to be transferred to the syringe before the usage. The usage of prefilled syringes minimizes drug waste and increases product life span.

The administration of drugs to patients, other than by an injection, has become more popular, due to the development of new drugs. Possible ways of administration are, in particular, oral, intranasal, topic, dermal and further external applications. For such applications, prefilled syringes are the best way to store, transport and administer the drugs.

Also, for mediums or drugs which are injected in the body, prefilled syringes are a convenient choice. In particular, modern vaccines react very sensible to outer influence factors and are therefore securely stored in a prefilled syringe.

The syringe bodies comprise a syringe barrel, a syringe tip and optionally a finger flange. In some cases, the syringe tip is already provided with a piercing means. A piercing means may be a needle, a canula or a similar element.

The syringe bodies are produced by a primary packaging producer under clean room conditions. After a sterilization, the syringe bodies are provided with a tip cap and placed in a so-called syringe nest. The tip cap usually is placed on the syringe tip and may also enclose a needle arranged in the syringe tip.

Such a syringe nest is delivered to a pharmaceutical company which fills the syringes with the medium to administer. After the filling process, a plunger or piston is introduced in the syringe barrel, by which the barrel is closed on its proximal side. The filled syringe is then placed in a secondary packaging.

The tip cap is necessary to seal the tip and optionally the needle. Usually, the tip caps are at least partially made of an elastic material. Due to the limited space for a syringe in the syringe nest, the tip caps are usually designed as small as possible. Such small sized tip caps are, however, inconvenient for the user to handle. Further, they might be a choking hazard for small children.

In some cases, the user needs to set the dosage before the actual administration of the medium, which may be done at a later point. For this purpose, the tip cap needs to be temporally removed. The reclosing of the syringe with the tip cap may result in an unwanted introduction of a portion of air in the syringe barrel. Such a portion of air may cause irritations by the patient. In particular in cases where the medium in the syringe barrel is higher-viscosity medium, the enclosed portion of air is difficult to remove.

From the state of the art document WO 2014 / 045336 A1 is known. Said document discloses a syringe packaging container with a container body for containing a syringe which has a syringe needle protection cap fitted to the front end of the outer tube of the syringe. The container body comprises a base plate and surrounding side walls. The container body further comprises a syringe holding section for holding the syringe and a cap removing section for removing the syringe needle protection cap from the outer tube by separating the outer tube and the syringe needle protection cap from each other. In an initial position, in which the syringe is equipped with the cap, the syringe is arranged diagonally upwards in the package. A movement of the syringe towards the base plate causes an engagement of the needle protection cap with the cap removing section and a separation of the needle protection cap from the outer tube.

The document US 2016 / 375 199 A1 discloses a boot remover for removing a protective needle boot from a drug delivery device. The boot remover is arranged to engage the protective needle boot when attached to the drug delivery device. The boot remover comprises a tab, extending in a distal direction, wherein the tab is engageable to a packaging of the drug delivery device.

The document US 2017 / 143 893 A1 discloses a packaging body for an auto-injector, which is equipped with a cap. The packaging body comprising a semicircular engaging part, which is arranged on a side of the packaging body. After the removal of the Autoinjector from the package it may be inserted into the semicircular engaging part for removing the cap.

Object of the invention is therefore to provide a system comprising a prefilled syringe and a package that overcomes the above-mentioned disadvantages. The invention is defined by the appended claims.

The problem is addressed by a package, suitable and intended for receiving a prefilled syringe, comprising a syringe barrel and a syringe tip, wherein the syringe tip is provided with a tip cap, wherein the package comprises a first receiving element, in which the tip cap is at least partially embeddable, wherein the first receiving element provides an engagement by which the tip cap is detachable from the syringe tip.

Such a package has the advantage that the tip cap remains in the package when the syringe is retrieved from the package and may advantageously not be removed from the package. The tip cap is therefore inextricably integrated in the package and may not be perceived as a separate element besides the package. Thus, the tip cap does not constitute a choking hazard for children.

Further, the handling of the syringe is easier for the user, since the comparatively small tip cap may not be grasped and removed. Instead, a retrieval of the syringe from the package, which holds the entire syringe, already detaches the tip cap from the syringe tip.

The user is therefore presented only with an already opened syringe and does not have the opportunity to reseal the syringe with this tip cap. If a resealing is necessary, a suitable replacement cap may advantageously be used.

In the following, the expressions "distal" and "proximal" are understood such that a distal end of a syringe is closer to the application site as the proximal end. The expressions "distal direction" and "proximal directed" are understood analogously.

In the following, the longitudinal axis (X), horizontal axis (Y) and vertical axis (Z) are used in relation to the package body. The longitudinal axis (X'), horizontal axis (Y') and vertical axis (Z') are used in relation to the prefilled syringe. Further, the distal direction (X') and the proximal direction (Y') are used. The longitudinal axis (X"), horizontal axis (Y") and vertical axis (Z") are used in relation to the replacement cap. Further, the distal direction (X') and the proximal direction (Y') are used.

According to a preferred embodiment, the package comprises a package body forming a first cavity defined by a sidewall and a bottom wall. Preferably, the prefilled syringe is arrangeable in said first cavity. Preferably, the package body extends along a longitudinal axis (X), a horizontal axis (Y) and a vertical axis (Z), which are perpendicular to each other.

According to a preferred embodiment, at least one second receiving element is provided in the first cavity of the package body, in which the syringe barrel is receivable. Preferably two second receiving elements are provided in the first cavity of the package body. Preferably, the at least one second receiving element is arranged on the bottom wall. It is further advantageous that the at least one second receiving element comprises a semicircular top section, arranged on a base section. The semicircular top section extends preferably along the horizontal axis (Y) and the longitudinal axis (X) and is open in the vertical axis (Z). Further is suitable and intended for receiving the syringe barrel. The semicircular top section comprises two arms which encompass the syringe barrel at least in part.

It is advantageous that a first of the second receiving elements is located close or at the region where the syringe barrel merges into the syringe tip. Further, it is preferred that the second of the second receiving elements is located close to the finger flange of the syringe arranged in the package body.

According to a further preferred embodiment, at least one third receiving element is provided in the first cavity of the package body, in which a rod of the prefilled syringe is receivable. Preferably, the third receiving element comprises two walls extending along the vertical axis (Z), forming a cavity in which the rod is received. Preferably, said walls extend along the longitudinal axis (X). The length of said walls is essentially the length of the rod. Advantageously the at least one third receiving element has a bottom wall which is formed semicircularly and connects said walls with each other. It is further advantageous that a gap between the second of the second receiving elements and the third receiving element is provided. Through this gap, the finger flange of the prefilled syringe may project, when the syringe is placed inside the package body. In other words, the finger flange is preferably sandwiched between the second of the second receiving elements and the third receiving element. Such a gap may fasten the prefilled syringe due to an engagement of the finger flange with the second of the second receiving elements and the third receiving element. The length of said walls is essentially determined by the length of a section of the rod, which projects from the syringe barrel without the length of the rod in said gap between the second of the second receiving elements and the third receiving element.

According to a further preferred embodiment, the bottom wall of the package body is provided with a base element, above which the first receiving element is arranged at least in part. Advantageously, the base element and the first receiving element are adjacent to said second receiving element, preferably to said first of the second receiving elements, in part. The base element and the first of the second receiving elements may preferably be formed as one element. Further, the base element preferably extends along the vertical axis (Z) and provides hereby a raise of the bottom wall along the vertical axis (Z). The upper surface of the base element preferably extends parallel to the bottom wall of the package body. The upper surface of the base element preferably extends preferably in a height L1 above the bottom wall of the package body.

According to a further preferred embodiment, at least one fourth receiving element is provided in the first cavity of the package body, in which a replacement cap for the prefilled syringe is receivable. Preferably, an opening of the inner cap is oriented along the longitudinal axis (X) of the package body or along the vertical axis (Z) of the package body.

According to a further preferred embodiment, the package body comprises a second cavity. Preferably, the second cavity is adjacent to the first cavity of the package body along the longitudinal axis (X) of the package body. Preferably, a replacement cap for the prefilled syringe is arranged in said second cavity. Preferably, an opening of the inner cap is oriented along the vertical axis (Z) of the package body.

According to the present invention, the first receiving element comprises an engagement wall with an elongated slot. Said elongated slot is extending along a vertical axis (Z) of the package body. The width of the elongated slot is larger than an outer diameter of the syringe tip, such that the syringe tip may project through the elongated slot. Preferably, the elongated slot comprises an upper end portion and a lower end portion, wherein the upper end portion is arranged along the vertical axis (Z) above the lower end portion. Advantageously, the upper end portion is designed semicircularly and a radial outer surface of the syringe tip abuts the semicircular end portion in a condition when the syringe is arranged in the package body.

According to the invention, the width of the elongated slot is smaller than an outer diameter of the tip cap. An inner surface of the engagement wall of the first receiving element adjacent to the elongated slot configured to abut a proximal end face of the tip cap, as a result of which the engagement of the tip cap with the receiving element is constituted. Advantageously, such an abutment may already exist in a state where the prefilled syringe is in a storage condition. Alternatively, the abutment and the following engagement may be induced by a relative movement of the prefilled syringe to the first receiving element or the engagement wall. Such a relative movement may preferably be induced by a retrieving movement of the prefilled syringe, wherein the engagement wall stays preferably stationary. Alternatively, such a relative movement may advantageously be induced by a movement of the first receiving element or the engagement wall, wherein the prefilled syringe stays preferably stationary.

Preferably, the elongated slot is placed along the vertical axis (Z) above the base element. According to a further preferred embodiment, the syringe is received by the preferably two second receiving elements. Thus, in a state where the syringe is in the package body, a central axis of the syringe extends preferably parallel to the bottom wall of the package body. Advantageously, the central axis extends in a height L2 above the bottom wall. In the section of the syringe tip, the central axis extends preferably in a height L3 above the base element. The height L3 is by the height L1 smaller than the height L2.

According to a further preferred embodiment, the first receiving element comprises a concave shaped or hollow body, which is preferably formed by a sidewall and a top wall. Preferably, the hollow body does not comprise a bottom wall and is, therefore, open on the lower side along the vertical axis (Z). Advantageously, the engagement wall is part of said sidewall of the concave shaped or hollow body. It is further favorable that the concave shaped, or hollow body which is preferably a separate element, is connected to the base element of the package body. Such a connection could be a clamping connection, a snap connection, a welded connection or an adhesion bond, or similar connections.

It is also conceivable that the first receiving element comprises a hollow body which is preferably formed by a sidewall and a top wall and a bottom wall. Preferably, the bottom wall is connected to the base element of the package body. Such a connection could be a clamping connection, a snap connection, a welded connection or an adhesion bond, or similar connections.

In order to retrieve the syringe from the package, the syringe may preferably be tilted. The pivot of such a rotation is advantageously the support point of the syringe barrel on the first of the second receiving elements. A proximal section of the syringe, which is essentially the syringe barrel, may then be moved in a direction away from the bottom wall of the package body. The syringe tip, which is on the opposite side of the pivot, moves preferably in the elongated slot towards the upper surface of the base element. The maximal angle a, in which the syringe may rotate, is preferably set by the distance between an outer surface of the tip cap and the base element.

Preferably, after tilting the syringe relative to the package by at least an angle a, the syringe is retrievable by a pulling movement, which is essentially along an axis extending in the tilting angle. By such a pulling movement, the proximal face of the tip cap engages advantageously with the inner surface of the engagement wall of the first receiving element adjacent to the elongated slot. By a further pulling movement, the tip cap is detached from the syringe tip and remains in the cavity formed by the concave shaped or hollow body and the base element.

The tilting angle α is preferably in a range between 10° and 75°, more preferably in a range between 20° and 60°, more preferably in a range between 30° and 55°, more preferably between 40° and 50°.

According to a further preferred embodiment, the sidewall extends essentially along a vertical axis (Z). By "extending essentially" it is meant that the sidewall may have a comparatively small angle to the vertical axis (Z). Such an angle could be in range between 0° to 25°, preferably 0° to 10°, preferably 0° to 5°.

Advantageously, the sidewall of the package body is provided with a flange on its upper end, wherein the flange extends outwards essentially along the longitudinal or the horizontal axis. Preferably, the flange surrounds the package body.

According to a further preferred embodiment, the package comprises a closure element which covers the package body at least in part. Preferably, the closure element covers the package body entirely.

According to a further preferred embodiment, the closure element is connected to the flange, provided on the side walls. The closure element could be a foil or a dimensionally stable element. The closure element could be connected to the flange by a clamping connection, a snap connection, a welded connection or an adhesion bond, or similar connections. Preferably, before the usage, such a connection is torn off, preferably, before the usage in order to expose the prefilled syringe in the package body.

According to a further preferred embodiment, the closure element is slidably arranged on the package body. Preferably, the closure element is slidable along a slide direction, which is preferably oriented along the longitudinal direction (X). Advantageously, the closure element is provided with a guidance element, which extends along the slide direction, in particular, the longitudinal direction (X) on each edge extending in the direction of the closure element. Preferably, each flange of the two sidewalls extending in the slide direction, in particular, the longitudinal directions, is received in a guidance element.

According to a further preferred embodiment, the flange, which surrounds the package body, is provided with at least one latching element, preferably at least two latching elements. Advantageously, the at least one latching element engages in at least one latching recess of the closure element. Preferably, the at least one latching element is provided on the upper surface of the flange, surrounding the package body. It is further favorable that the closure element is provided with a plurality of latching recesses. Preferably, the at least one latching element engages the latching recess, following in the sliding direction, when the closure element slides along the sliding direction.

According to a further preferred embodiment, the first receiving element is formed by the closure element at least in part and the package body at least in part. Thus, the tip cap may preferably be embedded in the space limited by the closure element, the bottom wall and the side walls of the package body. According to a further preferred embodiment, the closure element comprises the engagement wall. The first receiving element is therefore preferably a hollow body which is defined by the closure element, in particular the engagement wall, and a top portion of the closure element, the bottom wall and the side walls of the package body. The first receiving element according to this embodiment is therefore not a separate element, but instead, it is formed by the package body and the closure element.

Advantageously, by a sliding movement of the closure element, the engagement of the tip cap with the receiving element is constituted. Thus, the tip cap is removable from the syringe tip by the engagement induced by the sliding movement of the closure element. Preferably, the closure element is slid along the longitudinal axis (X) in order to access the syringe.

According to a further preferred embodiment, the closure element is provided with a recess which extends along a longitudinal axis (X) of the package body and/or a longitudinal axis (X') of the prefilled syringe. Preferably, the length of the recess is essentially the length of the prefilled syringe. The length of the prefilled syringe is hereby the length of the syringe barrel, the rod protecting from the barrel and the syringe tip with the tip cap. Under the expression "essentially the length" it is understood that the length of the recess is at least the length of the syringe, as defined above, plus a minor tolerance surplus of the length, such that a convenient retrieval of the syringe through the recess is possible.

Preferably, the width of a distal section of the recess is essentially the width of the at least one second receiving element. Preferably, the width of a distal section of the recess is essentially the width of a semicircular top section width of the at least one second receiving element. Under the expression "essentially the width" it is understood that the width of a distal section is at least the width of the at least one second receiving element plus a minor tolerance surplus of the width, such that a convenient sliding movement of the closure element is possible.

According to a further preferred embodiment, a grasp section of the recess is suitable and intended for a user to grasp the prefilled syringe. Preferably, a grasp section of the recess extends along a horizontal axis (Y) of the package body and/or a longitudinal axis (Y') of the prefilled syringe, arranged in the package body. The grasp section of the recess has advantageously a length which allows a user to insert one or two fingers in the grasp section of the recess. The grasp section of the recess preferably comprises a sidewall which extends along the vertical axis (Z) towards the bottom wall of the package body. It is further favorable that the grasp section of the recess comprises a bottom wall extending parallel to the bottom wall of the package body, wherein the bottom wall of the grasp section is arranged at a height L5 above the bottom wall of the package body.

Further, it is advantageous that a flange section of the recess extends along a horizontal axis (Y) of the package body and/or a horizontal axis (Y') of the prefilled syringe. Preferably, the width along the horizontal axis (Y) of the package body and/or the horizontal axis (Y') of the prefilled syringe of a flange section along the recess is essentially the width of the finger flange of the prefilled syringe. Under the expression "essentially the width" is understood that the width of the recess is at least the width of the syringe plus a minor tolerance surplus of the width, such that a convenient retrieval of the syringe through the recess is possible.

According to a further preferred embodiment, the closure element is slidable from a closed position to an open position. Advantageously, in the open position of the closure element, the flange section of the recess is positioned along a vertical axis (Z) above the finger flange of the prefilled syringe arranged in the package body, as a result of which the prefilled syringe is retrievable from the package. Preferably, in the closed position, a blocking section of the closure element is positioned along a vertical axis (Z) above the finger flange as a result of which the prefilled syringe is not retrievable from the package. Preferably, the blocking section of the closure element is positioned along the longitudinal axis (X) between the grasp and the flange section of the recess. Advantageously, the blocking section of the closure element comprises two projections projecting along the horizontal axis in the recess of the closure element.

Advantageously, the user slides the closure element along the longitudinal axis (X) from the close position to the open position. It is favorable that, due to this sliding movement, the engagement wall engages the proximal face of the tip cap, as a result of which the tip cap is detached from the syringe tip. At the same time, the recess has essentially the contour of the syringe with an additional grasp section, which is suitable and intended to grasp the syringe. In the open position, the recess matches with the prefilled syringe in the package. The user may then retrieve the prefilled syringe without the tip cap. The tip cap remains in the cavity, limited by the package body and the closure element.

According to a further preferred embodiment, the closure element comprises at least one second latching element. Preferably, the at least one second latching element has a tongue-like shape and engages the package body. In particular, this engagement locks the open position, such that a sliding movement of the closure element back to the closed position is not possible. Thus, a reuse of the package is not possible and a tampering of the package is avoided. According to a further preferred embodiment, the package consists of plastic. Preferably, the package is produced in an injection molding process preferably by multi-component injection molding.

The object is addressed by a system comprising a prefilled syringe with syringe barrel and a syringe tip and a package suitable and intended for receiving the prefilled syringe, wherein the syringe tip is provided with a tip cap, wherein the tip cap is at least partially embedded in a first receiving element of the package, wherein the tip cap is detachable by an engagement of the tip cap with the receiving element from the syringe tip.

The system may comprise the single features or combinations of the features described above for the package. Also, the package may comprise the single features or combinations of the features described for the system. Further, the same advantages may apply for the system as described above for the package.

According to a preferred embodiment, the prefilled syringe further comprises a plunger or a piston, connected to a rod projecting out of the syringe barrel. Advantageously, the prefilled syringe comprises also a finger flange. The finger flange may be formed on the syringe barrel. It is also conceivable that a separate finger flange element is connected to the syringe barrel, which provides a larger gripping surface. The syringe tip is preferably formed as a cone. Advantageously, the syringe tip has a Luer-Cone. Currently, ISO 80369 governs the Luer standards. The syringe tip may preferably also be equipped with a piercing means which may be a needle, a canula or a similar means. The prefilled syringe is advantageously provided with a tip cap which is arranged on the syringe tip. The tip cap may consist at least in part of an elastomeric material. Advantageously the prefilled syringe consists of glass or plastics.

The prefilled syringe is provided with a tip cap which is arranged on the syringe tip. The tip cap may preferably consist at least in part of an elastomeric material.

According to a preferred embodiment, the first receiving element comprises an engagement wall with an elongated slot, extending along a vertical axis (Z) of the package body. Preferably, the width of the elongated slot is larger than an outer diameter of the syringe tip Advantageously, the syringe tip projects through the elongated slot. It is further favorable that the width of the elongated slot is smaller than an outer diameter of the tip cap.

According to a preferred embodiment, an abutment of a proximal end face of the tip cap with an inner surface of the engagement wall of the first receiving element adjacent to the elongated slot constitutes the engagement of the tip cap with the receiving element.

According to a further preferred embodiment, the first receiving element comprises a concave shaped or hollow body, which is preferably formed by a sidewall and a top wall. Preferably, the concave shaped body does not comprise a bottom wall and is, therefore, open on the lower side along the vertical axis (Z). Advantageously, the engagement wall is part of said sidewall of the concave shaped body. It is further favorable that the concave shaped body is connected to the base element of the package body. Such a connection could be a clamping connection, a snap connection, a welded connection or an adhesion bond, or similar connections.

It is also conceivable that the first receiving element comprises a hollow body which is preferably formed by a sidewall and a top wall and a bottom wall. The hollow body would advantageously be placed on the syringe before the syringe is placed in the package, such that the tip cap is completely enclosed by the concave shaped body. In a next step, preferably the syringe and the hollow body are placed in the package and the hollow body is connected to the base element.

In order to retrieve the syringe from the package, the syringe may preferably be tilted. The pivot of such a rotation is advantageously the support point of the syringe barrel on the first of the second receiving elements. A proximal section of the syringe, which is essentially the syringe barrel, may then be moved in a direction away from the bottom wall of the package body. The syringe tip, which is on the opposite side of the pivot, moves preferably in the elongated slot towards the upper surface of the base element. The maximal angle a, in which the syringe may rotate, is preferably set by the distance between an outer surface of the tip cap and the base element.

Preferably, after tilting the syringe relative to the package by at least an angle a, the syringe is retrievable by a pulling movement, which is essentially along an axis extending in the tilting angle. By such a pulling movement, the Z proximal face of the tip cap engages advantageously the inner surface of the engagement wall of the first receiving element adjacent to the elongated slot. By a further pulling movement, the tip cap is detached from the syringe tip and remains in the cavity formed by the concave shaped or hollow body and the base element.

According to a further preferred embodiment, the package comprises a closure element which covers the package body at least in part. Preferably, the closure element covers the package body entirely. Advantageously, the closure element is connected to a flange provided on the side walls. The closure element could be a foil or a dimensionally stable element. The closure element could be connected to the flange by a clamping connection, a snap connection, a welded connection or an adhesion bond, or similar connections. Preferably, before the usage, such a connection is torn off, preferably before the usage in order to expose the prefilled syringe in the package body.

According to a further preferred embodiment, the package comprises a closure element which covers the package body at least in part. Preferably, the closure element is slidably arranged on the package body. It is further advantageous that the closure element comprises the engagement wall. Further, it is favorable that the closure element is provided with a recess which has essentially the contour of the prefilled syringe with an additional grasp section, which allows the user to grasp the prefilled syringe. Under the expression "essentially the contour of the prefilled syringe" it is understood that the dimensions of the recess are at least the dimensions of the syringe plus a minor tolerance surplus of the dimensions, such that a convenient retrieval of the syringe through the recess is possible.

According to a further preferred embodiment, the closure element is slidable from a closed position to an open position. Advantageously, in the open position of the closure element the flange section of the recess is positioned along a vertical axis (Z) above the finger flange of the prefilled syringe arranged in the package body, as a result of which the prefilled syringe is retrievable from the package. Preferably, in the closed position a blocking section of the closure element is positioned along a vertical axis (Z) above the finger flange, as a result of which the prefilled syringe is not retrievable from the package. Preferably, the blocking section of the closure element is positioned along the longitudinal axis (X) between the grasp and the flange section of the recess. Advantageously, the blocking section of the closure element comprises two projections projecting along the horizontal axis in the recess of the closure element.

Advantageously, the user slides the closure element along the longitudinal axis (X) from the closed position to the open position. It is favorable that, due to this sliding movement, the first receiving element in particular the engagement wall engages the proximal face of the tip cap, as a result of which the tip cap is detached from the syringe tip and remains in the cavity defined by the first receiving element. In the open position, the recess matches with the prefilled syringe

in the package. The user may then retrieve the prefilled syringe without the tip cap. The tip cap remains in the cavity defined by the package body and the closure element.

According to a further preferred embodiment, the flange, which surrounds the package body, is provided with at least one latching element, preferably at least two latching elements. Advantageously, the at least one latching element engages in at least one latching recess of the closure element. Preferably, the at least one latching element is provided on the upper surface of the flange, surrounding the package body. It is further favorable that the closure element is provided with a plurality of latching recesses. Preferably, the at least one latching element engages the latching recess following in the sliding direction, when the closure element slides along the sliding direction.

According to a further preferred embodiment, the closure element comprises at least one second latching element. Preferably, the at least one second latching element has a tongue-like shape and engages on the package body. In particular, this engagement locks the open position, such that a sliding movement of the closure element back to the closed position is not possible. Thus, a reuse of the package is not possible, and a tampering of the package is avoided.

According to a further preferred embodiment, the system comprises a replacement cap for the prefilled syringe. Advantageously, the replacement cap comprises an inner cap for an engagement with the syringe tip. Preferably, the inner cap consists at least partially of an elastomeric material, which allows a sealing connection between the inner cap and the syringe tip.

Preferably, the replacement cap is produced by in an injection molding process preferably by multi-component injection molding.

It is further preferable that the replacement cap comprises an outer collar for mounting the replacement cap on the syringe barrel. It is also advantageous that the replacement cap comprises a grip portion for a user to grasp the replacement cap. Preferably, the replacement cap is arranged in the package.

In some cases, the prefilled syringe may be used more than once, or the user needs to set the dosage before the actual administration of the medium, which may be done at a later point. The prefilled syringe therefore needs to be closed by a cap. Since the original tip cap is no longer accessible to the user due to the inextricable integration of the tip cap in the package, a replacement cap is necessary. It is therefore advantageous that the replacement cap is already provided in the same package as the syringe.

According to a further preferred embodiment, the first cavity of the package body is provided with at least one forth receiving element, in which the replacement cap is received. Preferably, an opening of the inner cap is oriented along the longitudinal axis (X) of the package body or along the vertical axis (Z) of the package body.

An orientation along the longitudinal axis (X) bears the advantage that the collar of the replacement cap may be designed longer, since along the longitudinal axis (X) more space is available in the package body.

An orientation along the vertical axis (Z) of the package body bears the advantage that the user may insert the syringe in the replacement cap while the replacement cap is still arranged in the package body. A retrieval of the replacement cap before connecting it with the prefilled syringe is therefore not necessary. The user may therefore handle the syringe only with one hand.

According to a further preferred embodiment, the replacement cap is arranged in a second cavity of the package body. Preferably, the second cavity of the package body is adjacent to the first cavity of the package body along the longitudinal axis (X) of the package body. Advantageously, an opening of the inner cap is oriented along the vertical axis (Z) of the package body.

Preferably, the closure element covers in a closed position the second cavity entirely. In the open position, the closure element is removed from the second cavity.

Preferably, in a packaging process, the syringe is placed in the package, wherein the syringe barrel is supported by the second receiving elements, such that a central axis of the syringe extends preferably parallel to the bottom wall of the package body. Advantageously, the central axis of the prefilled syringe extends in a height L2 above the bottom wall of the package body and the syringe tip extends in a height L3 above the base element. Further, a preferred replacement cap may be placed in the package body.

If an embodiment is present where the preferred engagement wall is part of a concave shaped body, the concave shaped body is preferably placed on the base element in a next step, such that the tip cap is completely enclosed by the concave shaped body. The syringe tip is preferably enclosed partially by the concave shaped body. Eventually, the concave shaped body is connected to the base element by a suitable connection. Afterwards, the package body is closed with the closure element.

If an embodiment is present where the preferred engagement wall is part of a closure element, the closure element is arranged on the package body in a next step, such that the syringe tip is received in the preferred elongate recess and the engagement wall is placed along a longitudinal axis (X) behind the tip cap. An upper wall of the closure element, the engagement wall and further sidewalls of the closure element extending along a vertical axis (Z) towards the bottom wall of the package body define, together with the sidewalls and the bottom wall of the package body, a front compartment in which the tip cap remains inextricable after the detachment from the syringe tip.

The object is also addressed by a usage of a system according to the above-mentioned embodiments. The system comprises a prefilled syringe with syringe barrel and a syringe tip and a package suitable and intended for receiving the prefilled syringe, wherein the syringe tip is provided with a tip cap, wherein the tip cap is at least partially embedded in a first receiving element of the package, wherein the tip cap is detachable by an engagement of the tip cap with the receiving element from the syringe tip.

The use of the system may comprise the single features or combinations of the features described above for the package or the system. Also, the package or the system may comprise the single features or combinations of the features described for the use. Further, the same advantages may apply for the use as described above for the package or the sytem.

The usage comprises the following steps:
a) Optionally removing the closure element;
b) Inducing a relative movement between the prefilled syringe and the first receiving element, wherein the relative movement results in an engagement of the first receiving element with the tip cap and a detaching of the tip cap from the syringe tip, wherein the tip cap remains in the package.

According to a preferred embodiment the use comprises the step to set the dosage of the prefilled syringe.

According to one preferred embodiment, the relative movement is induced by a pulling movement of the prefilled syringe along an axis which is oriented in an angle α relative to the package body, wherein the angle α results from lifting the prefilled syringe, which induces a tilt of an angle α of the prefilled syringe relative to a package body.

According to a further preferred embodiment, the relative movement is induced by a sliding movement of the closure element, which comprises the first receiving element, wherein the sliding movement results in an engagement of an engagement of the first receiving element with the tip cap and a detaching of the tip cap from the syringe tip.

Further advantages, aims and properties of the present invention will be described by way of the appended drawings and the following description in the drawings:
- Fig. 1: shows a prefilled syringe without a tip cap;
- Fig. 2a: shows a prefilled syringe with a tip cap;
- Fig. 2b: shows a replacement cap;
- Fig. 3: shows a package body according to one embodiment without a prefilled syringe;
- Fig. 4: shows a package body according to one embodiment with a prefilled syringe and a replacement cap;
- Fig. 5: shows a package body according to one embodiment with a prefilled syringe and a replacement cap;
- Fig. 6: shows a package body according to one embodiment with a prefilled syringe and a replacement cap;
- Fig. 7a: shows a prefilled syringe with a tip cap;
- Fig. 7b: shows a replacement cap according to a further embodiment;
- Fig. 8: shows a package body according to a further embodiment without a prefilled syringe;
- Fig. 9: shows a package body according to a further embodiment with a prefilled syringe;
- Figs. 10: shows a closure element of a package;
- Figs. 11: shows a package body according to a further embodiment with a prefilled syringe;
- Figs. 12: shows a package body according to a further embodiment without a prefilled syringe;
- Figs. 13: shows a package body according to a further embodiment without a prefilled syringe.

In the following, the longitudinal axis (X), horizontal axis (Y) and vertical axis (Z) are used in relation to the package body (3). The longitudinal axis (X'), horizontal axis (Y') and vertical axis (Z') are used in relation to the prefilled syringe (100). The longitudinal axis (X"), horizontal axis (Y") and vertical axis (Z") are used in relation to the replacement cap (300). Further, the distal direction (X') and the proximal direction (Y') are used.

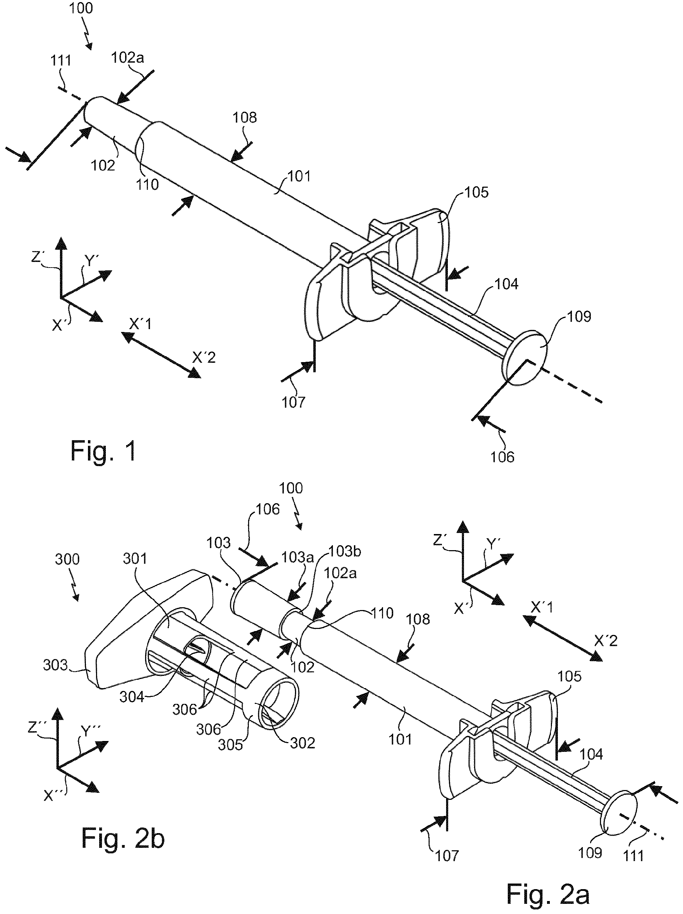

In figures 1, 2a and 7a, a prefilled syringe is displayed. A prefilled syringe (100) comprises a syringe barrel (101) and a syringe tip (102). The syringe barrel (101) is cylindrically shaped and open at its proximal end. At its distal end, the syringe barrel (101) merges along the distal direction (X'1) into the syringe tip (102). The syringe tip (102) may have the form of a cone. Advantageously, the syringe tip (102) is a Luer-cone. It is also conceivable that a piercing means, in particular a needle, a canula or a similar means, is arranged in the syringe tip (102). In the syringe barrel (101), the medium to administer is stored. On its proximal end, the syringe barrel (101) is closed with a plunger or a piston (not visible in the figures), which is movable in the syringe barrel (102). The plunger or the piston are attached to a rod which projects in the proximal direction (X'2) from the syringe barrel (101). On the proximal end of the rod (104), a finger support (109) is arranged. Further, the syringe barrel is provided with a finger flange (105). In this case, the finger flange is a clip-on-finger flange which may be clipped on a finger flange on the syringe barrel (101). Such clip-on-finger flanges bear the advantage of a larger area to grasp for the user.

The prefilled syringe (100) is provided with a tip cap (103) which is arranged on the syringe tip (102). The tip cap (103) may consist at least in part of an elastomeric material.

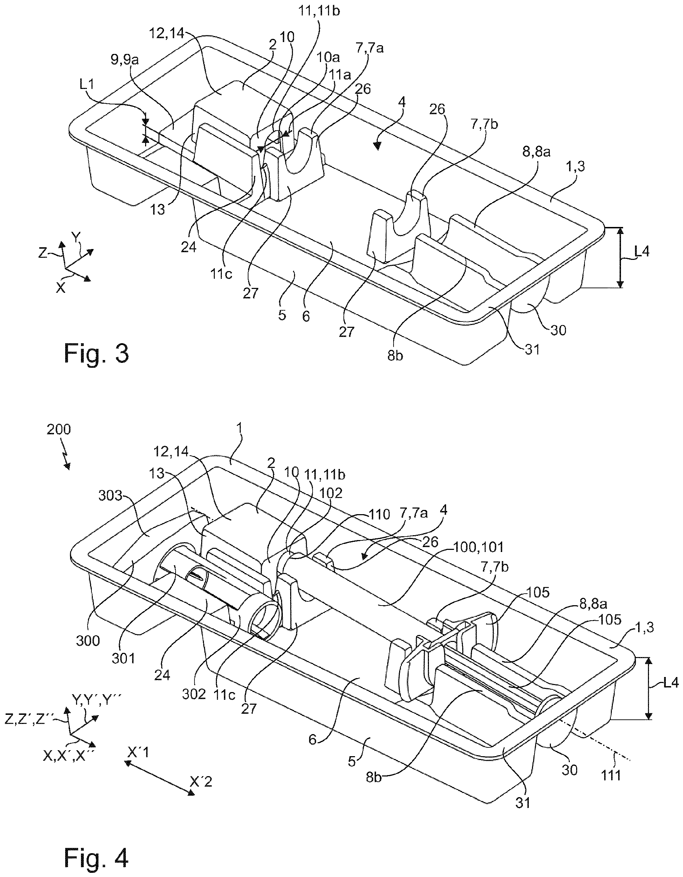

In the figures 3 to 6 and 8 to 13 a package (1) and/or a system (200) are depicted.

The package (1) is suitable and intended for receiving a prefilled syringe (100), comprising a syringe barrel (101) and a syringe tip (102), wherein the syringe tip (102) is provided with a tip cap (103), wherein the package (1) comprises a first receiving element (2), in which the tip cap (103) is at least partially embeddable, wherein the first receiving element (2) provides an engagement by which the tip cap (103) is detachable from the syringe tip (102).

The system (200) comprises a prefilled syringe (100) with syringe barrel (101) and a syringe tip (102) and a package (1) suitable and intended for receiving the prefilled syringe (100), wherein the syringe tip (102) is provided with a tip cap (103), wherein the tip cap (103) is at least partially embedded in a first receiving element (2) of the package (1), wherein the tip cap (103) is detachable by an engagement of the tip cap (103) with the receiving element (2) from the syringe tip (102).

The package (1) comprises a package body (3), forming a first cavity (4) defined by a sidewall (5) and a bottom wall (6), wherein the prefilled syringe (100) is arrangeable or arranged in said first cavity (4). The package body (3) extends along a longitudinal axis (X), a horizontal axis (Y) and a vertical axis (Z), which are perpendicular to each other. Further, the package body has a rectangular shape.

In the first cavity (4) of the package body (4), at least one second receiving element (7, 7a, 7b) is provided in the first cavity (4) of the package body (4), in which the syringe barrel (100) is receivable. The embodiments shown in the figures have two second receiving elements (7, 7a, 7b), which are arranged at the bottom wall (6) of the package body (3).

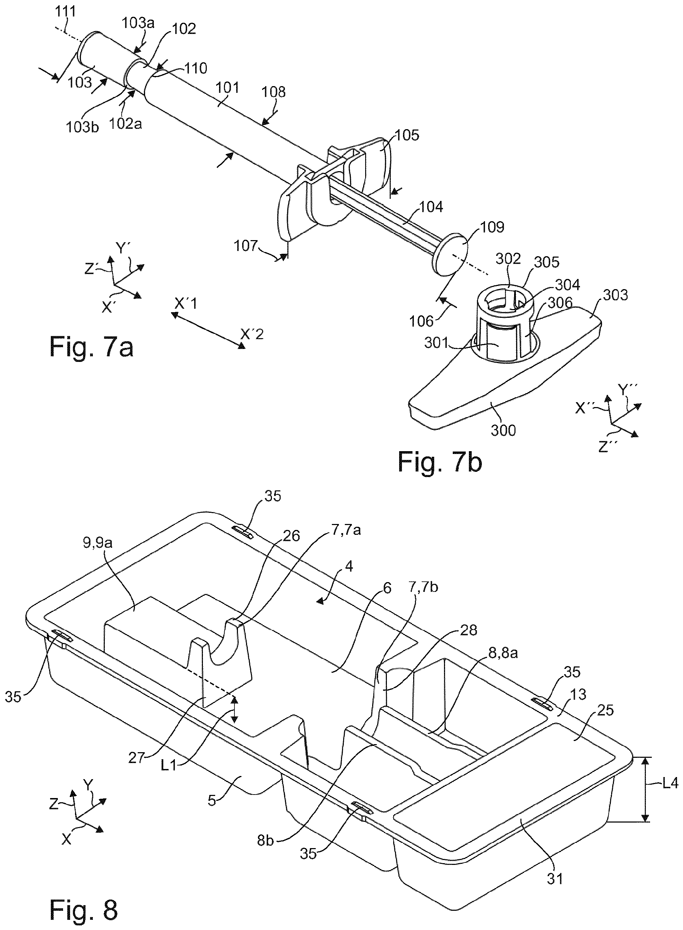

The first (7a) of the second receiving elements (7) comprises a semicircular top section (26) arranged on a base section (27). The semicircular top section (26) is dimensioned to receive the syringe barrel (101). According to the embodiments in figures 3 to 6, the second (7b) of the second receiving elements (7) also comprises a semicircular top section (26) arranged on a base section (27). According to the embodiments in figures 8 to 13, the second (7b) of the second receiving elements (7) is formed as a relatively wide semicircular section (28), where the syringe barrel (101) projects through. In case the prefilled syringe (100) is arranged in the package (1), the first (7a) of the second receiving elements (7) is located close or at the region (110) where the syringe barrel (101) merges into the syringe tip (102).

Further, the second (7b) of the second receiving elements (7) is located close to the finger flange (105) of the prefilled syringe (100), arranged in the package body (3). The first cavity (4) of the package body (3) is provided with a third receiving element (8) in which the rod (104) of the prefilled syringe (100) is receivable or received. The third receiving element (8) comprises two walls (8a, 8b) extending along the vertical axis (Z), forming a cavity in which the rod (105) is receivable or received. Further, the third receiving element (8) has a bottom wall formed semicircularly and connecting the walls (8a, 8b). The third receiving element (8) ends at its proximal end in a fixture element (30) in which the finger support (109) of the rod (104) is received.

Between the second of the second receiving elements (7, 7b) and the third receiving element (8), a gap (29) is formed. Through this gap (29), the finger flange (105) of the prefilled syringe (100) may project, when the prefilled syringe (100) is placed inside the package body (3). Thus, the finger flange (105) is sandwiched between the second (7b) of the second receiving elements (7) and the third receiving element (8), such that the prefilled syringe (100) is fastened in the package body (3).

The third receiving element (8) stretches, after the gap (29), essentially along the length of the section of the rod (104) projecting from the syringe barrel (101).

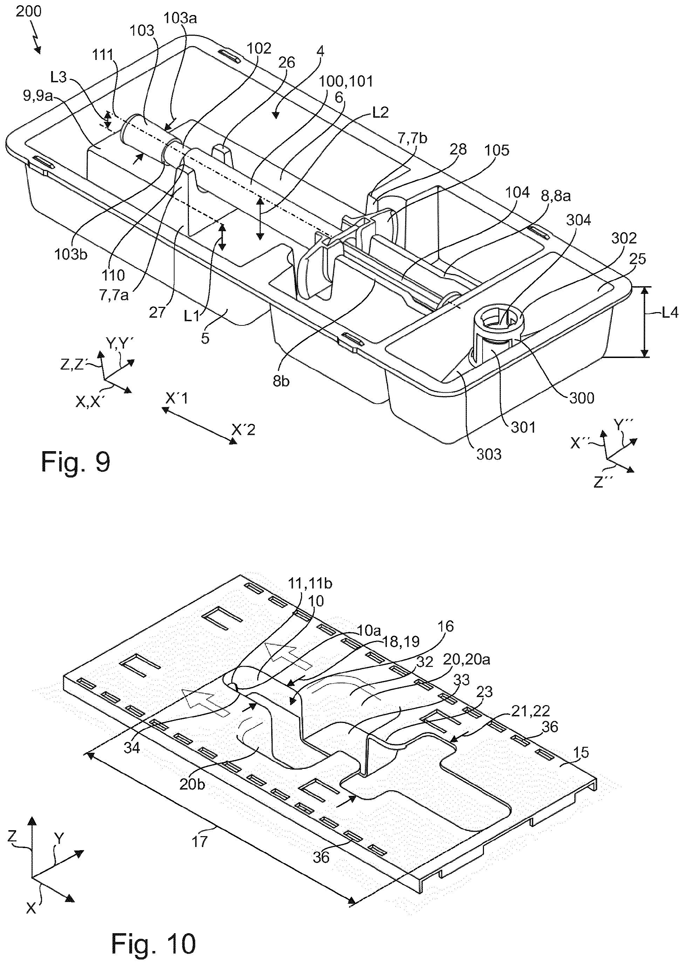

Further, the bottom wall (6) of the package body (3) is provided with a base element (9) above which the first receiving element (2) is arranged at least in part. The base element (9) is adjacent to the first (7a) of the second receiving elements (7). The base element (9) and the first (7a) of the second receiving elements (7) may even be formed as one element. This may, for example, be seen in figures 8 and 9. Further, the base element preferably extends along the vertical axis (Z) and provides hereby a raise of the bottom wall (6) of the package body (3) along the vertical axis (Z). The upper surface (9a) of the base element preferably extends parallel to the bottom wall of the package body. The upper surface (9a) of the base element (9) extends in a height L1 above the bottom wall (6) of the package body (3).

When the prefilled syringe (100) is placed in the package body (3), it is supported by the first (7a) of the second receiving elements (7), preferably by the second (7b) of the second receiving elements (7), and preferably by the third receiving element (8), in particular by the fixture element (30). Thus, in a state where the prefilled syringe (100) is in the package body (3), a central axis (111) of the prefilled syringe (100) extends parallel to the bottom wall (6) of the package body (3). The central axis (111) extends in a height L2 above the bottom wall (6) of the package body (3). In the section of the syringe tip (102), the central axis (111) extends preferably in a height L3 above the base element (9). The height L3 is by the height L1 smaller than the height L2.

The side walls (5) of the package body (3) surround the package body (3) along the longitudinal axis (X) and the horizontal axis (Y). The sidewalls (5) extend along the vertical axis (Z) with a height L4. The vertical axis (Z) upper end of the side walls is provided with a flange (31). The flange extends outwards and surrounds the package body (3) along the longitudinal axis (X) and the horizontal axis (Y), preferably without any gap.

The first receiving element (2) comprises an engagement wall (10) with an elongated slot (11), extending along a vertical axis (Z) of the package body (3). The width (11a) of the elongated slot (11) is larger than an outer diameter (102a) of the syringe tip (102), such that the syringe tip (102) may project or projects through the elongated slot (11).

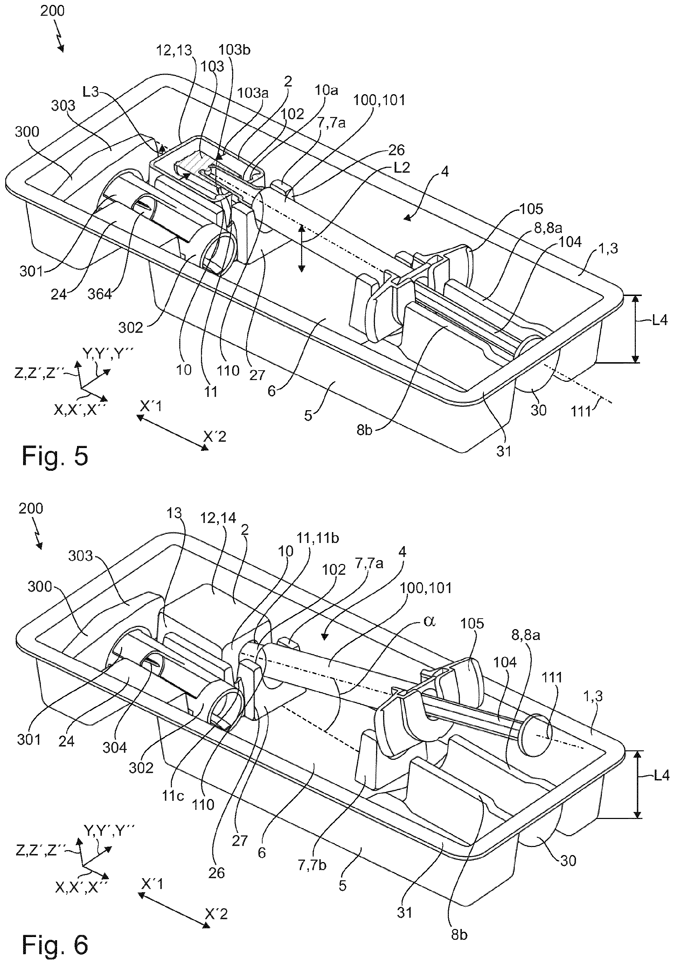

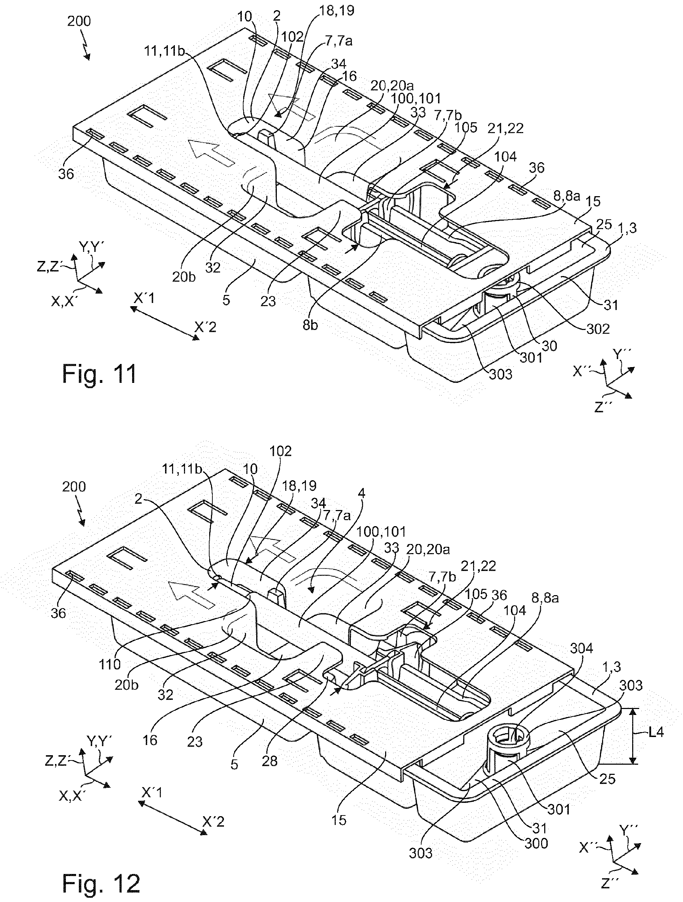

The width (11a) of the elongated slot (11) is smaller than an outer diameter (103a) of the tip cap (103). An inner surface (10a) of the engagement wall (10) of the first receiving element (2), which is adjacent to the elongated slot (11), may abut a proximal end face (103b) of the tip cap (103). As a result of the abutment, the engagement of the tip cap (103) with the receiving element (2) is constituted. Such an abutment may exist already in a state where the prefilled syringe (100) is in a storage condition. Alternatively, the abutment and the following engagement may be induced by a relative movement of the prefilled syringe (100) to the engagement wall (10). Such a relative movement may be induced by a retrieving movement of the prefilled syringe (100), wherein the engagement wall (10) stays stationary (displayed, for example, in figure 6). Alternatively, such a relative movement may be induced by a movement of the first receiving element (2) or the engagement wall (10), wherein the prefilled syringe (100) stays stationary (displayed, for example, in figures 11 to 13).

The elongated slot (11) comprises an upper end portion (11b) and a lower end portion (11c), wherein the upper end portion (11b) is arranged along the vertical axis (Z) above the lower end portion (11c). The upper end portion (11b) is designed semicircularly. Preferably, the radial outer surface of the syringe tip (102) abuts the semicircular upper end portion (11b) in a condition when the prefilled syringe (100) is arranged in the package body (3).

The embodiment according to figures 3 to 6 displays that the first receiving element (2) comprises a concave shaped or hollow body (12) formed by a sidewall (13) and a top wall (14). The engagement wall (10) is part of the sidewall (13) of the concave shaped body (12). Further, the concave shaped body (12) is connected to the base element (9) of the package body (3). Preferably, the concave shaped hollow body (12) does not comprise a bottom wall and is, therefore, open on the lower side along the vertical axis (Z). The engagement wall (10) is hereby part of said sidewall (13) of the concave shaped or hollow body (12). The concave shaped or hollow body (12) is further connected by a suitable connection to the base element (9) of the package body (3).

When the concave shaped or hollow body (12) is connected to the base element (9) of the package body (3), the tip cap (103) is entirely enclosed in a cavity formed by the concave shaped or hollow body (12) and the upper surface (9a) of the base element (9).

The package body (3) according to the embodiments in figures 3 to 6 may also be closed by a suitable closure element which covers the package body at least in part. Preferably, the closure element covers the package body entirely.

Such a closure element is connected by a suitable connection to the flange (31), provided on the side walls (5) of the package body (3). The closure element could be a foil or a dimensionally stable element.

In figure 6, a retrieval of the prefilled syringe (100) from the package (1) is displayed. The syringe is lifted such that it tilts. The pivot of such rotation is the support point of the syringe barrel (101) on the first (7a) of the second receiving elements (7). The pivot is in between a proximal section of the prefilled syringe (100), which is essentially the syringe barrel (101) and the syringe tip (102) with preferably the region (110) where the syringe barrel merges into the syringe tip (102). The proximal section of the prefilled syringe (100) may be moved in a direction away from the bottom wall (6) of the package body (3), as a result of which the syringe tip (102), which is on the opposite side of the pivot, moves in the elongated slot (11) downwards towards the upper surface (9a) of the base element (9). The maximal angle a, in which the prefilled syringe (100) may rotate, is preferably set by the distance between an outer surface of the tip cap (103) and the upper surface (9a) of the base element (9). Such an angle α may be enclosed by the bottom wall (5) of the package body (3) and the central axis (111) of the prefilled syringe (100).

After tilting the syringe relative to the package body (3) by at least an angle a, the prefilled syringe (100) is retrievable by a pulling movement, which is essentially along the central axis (111) of the prefilled syringe (100), which is tilted by an angle α relative to the bottom wall (5). By such a pulling movement, the proximal face (103b) of the tip cap (103) engages the first receiving element (2), in particular the inner surface (10a) of the engagement wall (10), which is adjacent to the elongated slot (11). By a further pulling movement, the tip cap (103) is detached from the syringe tip (102) and remains in the cavity formed by the concave shaped or hollow body (12) and the upper surface (9a) of the base element (9).

The system (200) comprises a replacement cap (300) for the prefilled syringe (100). The replacement cap (300) comprises an inner cap (301) for an engagement with the syringe tip (102). The inner cap (301) may consist at least partially of an elastomeric material, such that a sealing connection to the syringe tip (102) is possible. The syringe tip (102) may be introduced in the inner cap (301) through the opening (304) of the inner cap (301).

Further, the replacement cap (300) comprises an outer collar (302) for mounting the replacement cap (300) and a grip portion (303) for a user to grasp the replacement cap (300). The grip portion (303) has a rectangular shape.

The outer collar (300) is formed as a ring (305) which is arranged along a longitudinal axis (X") above the opening (304) of the inner cap (301). The ring (305) is supported by three struts (306), which are arranged at the grip portion (303) and surround the inner cap (301) radially. The struts (306) project from the ring (305) radially inward and abut at the syringe barrel (101) when the replacement cap (300) is placed on the prefilled syringe (100).

In the embodiments according to figures 3 to 6, the first cavity (4) of the package body (3) is provided with a forth receiving element (24), in which the replacement cap (300) is received. An opening (304) of the inner cap (301) is oriented along the longitudinal axis (X) of the package body (3) of the package body (3).

The forth receiving element (24) has two walls (24a, 24b) which extend along the longitudinal axis (X) and along the vertical axis (Z). Between the two walls (24a, 24b), the collar (302) of the replacement cap (300) is placed. A first wall (24a) is formed integrally in a sidewall (5) of the package body (3). The second wall (24b) is formed along the horizontal axis (Y) adjacent to the base element (9), the concave shaped body (12) and the first (7a) of the second receiving elements (7). The grip element is sandwiched between the concave shaped body (12) and a sidewall (5) of the package body, extending along the horizontal axis (Y).

The embodiments according to figures 8 to 13 display a package (1) comprising a closure element (15), which covers the package body (3) at least in part, wherein the closure element (15) is slidably arranged on the package body (3). The first receiving element (2) comprises the closure element (15) at least in part and the package body (3) at least in part. In particular, the first receiving element (2) comprises the bottom wall (6) and the sidewalls (5) of the package body (3). This closure element (15) comprises the engagement wall (10). The first receiving element (2) is therefore also defined by the engagement wall (10) and the contact to the base element (9). In a vertical direction (Z), the first receiving element (2) is defined by a top wall of the closure element (15). Similar to the embodiments according to figures 3 to 6, also here, a hollow body is formed. In contrast to these embodiments, the first receiving element (2) is not a separate element, but instead, it is formed by the package body (3) and the closure element (15).

Thus, by a sliding movement of the closure element (15), the engagement of the tip cap (103) with the receiving element (2) is constituted. The tip cap (103) is, therefore, detachable from the syringe tip (102) by the engagement induced by the sliding movement along the longitudinal axis (X) of the closure element (15).

The closure element (15) is provided with a recess (16) which extends along the longitudinal axis (X) of the package body (3) and/or the longitudinal axis (X') of the prefilled syringe (100). The length (17) of the recess (16) is essentially the length (106) of the prefilled syringe (100).

The width (19) of a distal section (18) of the recess (16) is essentially the width of the at least one second receiving element (7, 7a), in particular, the width of a semicircular top section (26) of the first (7a) of the second receiving elements (7). The width of the semicircular top section (26) is the width (108) of the syringe barrel (102) plus the width of each arm of the semicircular top section (26).

The engagement wall (10) extends along the vertical axis (Z) towards the upper surface (9a) of the base element (9). Preferably, the engagement wall (10) contacts the upper surface (9a) of the base element (9). Further, the engagement wall (10) comprises two lateral sidewalls (34) extending along the two lateral sides of the distal section (18) along the longitudinal axis (X). A sliding movement of the closure element (15) from a closed position to an open position results in an enclosure of the semicircular top section (26) of the first (7a) of the second receiving elements (7) inside the two lateral sidewalls (34) of the engagement wall (10). On a distal end of the distal section (18) the engagement wall (10) extends along a horizontal axis (Y) along the width of the distal section (18).

The recess further comprises a grasp section (20), which is suitable and intended for a user to grasp the prefilled syringe (100) and a flange section (21). The grasp section (20) is located along a horizontal axis (X') on both sides of the syringe barrel (101) such that the syringe barrel (101) is accessible. Thus, the syringe barrel (101) is positioned between the two halves (20a, 20b) of the grasp section (20). Each half (20a, 20b) is basically rectangular shaped, wherein a sidewall facing the syringe barrel (101) and a top wall are missing. Thus, each half (20a, 20b) comprises a sidewall (32), which extends along the vertical axis (Z) towards the bottom wall (6) of the package body (3), and a bottom wall (33) extending parallel to the bottom wall (6) of the package body (3). The bottom wall (33) of the grasp section (20) is arranged at a height L5 above the bottom wall (5) of the package body (3). Further, the bottom wall (33) of the two halves (20a, 20b) of the grasp section (20) are separated by a gap.

The flange section (21) of the recess (16) extends along the horizontal axis (Y) of the package body (3) and/or the horizontal axis (Y') of the prefilled syringe (100). A width (22) of a flange section (21) of the recess (16) is essentially the width (107) of the finger flange (105) of the prefilled syringe (100).

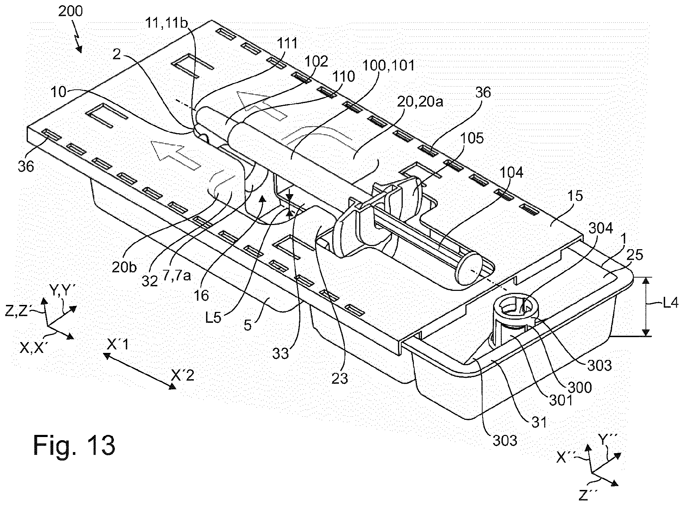

The closure element (15) is slidable from a closed position to an open position. In the open position of the closure element (15), the flange section (21) of the recess (16) is positioned along a vertical axis (Z) above the finger flange (105) of the prefilled syringe (100) arranged in the package body (3). As a result of which the prefilled syringe (100) is retrievable from the package body (3). This is displayed in figures 12 and 13. In the closed position, a blocking section (23) of the closure element (15) is positioned along a vertical axis (Z) above the finger flange (105). Thus, the prefilled syringe (100) is not retrievable from the package body (3), due to an abutment of the finger flange (105) with the blocking section (23). The blocking section (23) of the closure element (15is positioned along the longitudinal axis (X) between the grasp section (20) and the flange section (21) of the recess (16) and comprises two projections projecting along the horizontal axis (Y) into the recess (16) of the closure element (15).

In the embodiment according to the figures 8 to 13, the package body (3) comprises a second cavity (25), which is adjacent to the first cavity (4) of the package body (3) along the longitudinal axis (X) of the package body (3) and has preferably the same width as the first cavity (4). The replacement cap (300) is arranged in a second cavity (25) of the package body (3), wherein an opening (304) of the inner cap (301) is oriented along the vertical axis (Z) of the package body (3).

In order to retrieve the prefilled syringe (100) from the package body (5), the user slides the closure element (15) along the longitudinal axis (X) from the closed position to the open position. Due to this sliding movement, first receiving element (2) in particular the engagement wall (10) engages the proximal face (103b) of the tip cap (103). As a result of the engagement, the tip cap (103) is detached from the syringe tip (102) and is confined in the first receiving element (2) defined by the closure element (15) and the package body (3): In the open position, the recess (16) of the closure element (15), which has essentially the contour of the prefilled syringe (100) with an additional grasp section (20), matches with the prefilled syringe (100) in the package body (3). The user may then retrieve the prefilled syringe (100) without the tip cap (103). The tip cap (103) remains in the cavity defined by first receiving element, is delimited by the closure element (15) and the package body (5). In particular, this cavity is defined by the top wall of the closure element (15), the engagement wall (10), the side walls (20a) of the grasp section (20), the bottom wall (5) of the package body (3), and the side walls (5) of the package body (3).

The flange (31), which surrounds the package body (3), is provided with at least one first latching element (35), preferably at least two first latching elements (35), more preferably four first latching elements (35), wherein the at least one latching element (35) is provided on the upper surface of the flange (31). The at least one latching element (35) engages in at least one latching recess (36) of the closure element (15). The closure element (15) is provided with a plurality of latching recesses (36). In a sliding movement of the closure element (15), the at least one latching element disengages from one latching recess (36) and engages in (the along the longitudinal axis (X)) following the latching recess (36).

The closure element (15) comprises at least one second latching element (37), which has a tongue-like shape and engages on the package body. Preferably, the closure element (15) comprises at least two second latching elements (37), more preferably four second latching elements (37). An engagement of the second latching elements (37) locks the open position, such that a sliding movement of the closure element (15) back to the closed position is not possible. Thus, a reuse of the package is not possible, and a tampering of the package is avoided. Preferably, two of the second latching elements (37) engage the second (7b) of the second receiving elements (7) and the preferably other two of the second latching elements (37) engage a sidewall (5) of the package body (3) extending along the horizontal axis (Y) at the distal end of the package body.

All the features disclosed in the application documents are claimed as being essential to the invention if, individually or in combination, they are novel over the prior art.

### List of reference numerals

- 1: package
- 2: first receiving element;
- 3: package body;
- 4: first cavity of the package body;
- 5: sidewall of the package body
- 6: bottom wall of the package body
- 7: second receiving element
- 7a: first of the second receiving elements
- 7b: second of the second receiving elements
- 8: third receiving element
- 8a, 8b: walls of the third receiving element;
- 9: base element
- 9a: upper surface of the base element
- 10: engagement wall
- 10a: inner surface of the engagement wall
- 11: elongated slot
- 11a: width of the elongated slot
- 11b: upper end portion of the elongated slot
- 11c: lower end of the elongated slot
- 12: concave shaped body
- 13: sidewall of the concave shaped body
- 14: top wall of the concave shaped body
- 15: closure element
- 16: recess of the closure element
- 17: length of the recess
- 18: distal section
- 19: width of the distal section
- 20: grasp section of the recess
- 20a: two halves of the grasp section
- 21: flange section of the recess
- 22: width of a flange section
- 23: blocking section
- 24: fourth receiving element
- 25: second cavity of the package body
- 26: semicircular top section of the second receiving element
- 27: base section of the second receiving element
- 28: semicircular section of the second receiving element
- 29: gap between the second receiving elements and the third receiving element
- 30: fixture element
- 31: flange
- 32: sidewall of the grasp section
- 33: bottom wall of the grasp section
- 34: lateral sidewalls of the engagement wall
- 35: first latching element
- 36: latching recess
- 37: second latching element

- 100: prefilled syringe
- 101: syringe barrel
- 102: syringe tip
- 102a: outer diameter of the syringe tip
- 103: tip cap
- 103a: outer diameter of the tip cap
- 103b: distal end face of the tip cap
- 104: rod
- 105: finger flange
- 106: the length of the prefilled syringe
- 107: width of the finger flange
- 108: width of the syringe barrel
- 109: finger support
- 110: region where the syringe barrel merges in to the syringe tip
- 111: central axis of the prefilled syringe
- 200: system
- 300: replacement cap
- 301: inner cap
- 302: collar
- 303: grip portion
- 304: opening of the inner cap
- 305: ring
- 306: struts

- X: longitudinal axis of the package body
- Y: horizontal axis of the package body
- Z: vertical axis of the package body
- X': longitudinal axis of the prefilled syringe
- Y': horizontal axis of the prefilled syringe
- Z': vertical axis of the prefilled syringe
- X": longitudinal axis of the replacement cap
- Y": horizontal axis of the replacement cap
- Z": vertical axis of the replacement cap
- X'1: distal direction
- X'2: proximal direction
- L1: height of the upper surface of the base element above the bottom wall
- L2: height of the central axis of the syringe above the bottom wall
- L3: height of the central axis in the section of the syringe tip above the base element
- L4: height of the sidewall of the package body
- L5: height of the grasp section is arranged above the bottom wall of the package body
- α: angle

## Claims

1. System (200) comprising a prefilled syringe (100) with a syringe barrel (101) and a syringe tip (102) and a package (1) suitable and intended for receiving the prefilled syringe (100), wherein the syringe tip (102) is provided with a tip cap (103),
**characterized in that**
the tip cap (103) is at least partially embedded in a first receiving element (2) of the package (1), wherein the tip cap (103) is detachable by an engagement of the tip cap (103) with the first receiving element (2) from the syringe tip (102), wherein the first receiving element (2) comprises an engagement wall (10) with an elongated slot (11) extending along a vertical axis (Z) of the package body (3), wherein the width (11a) of the elongated slot (11) is smaller than an outer diameter (103a) of the tip cap (103), wherein the receiving element (2) forms a hollow body, wherein the tip cap (103) remains in a cavity of the hollow body after being detached from the syringe tip (102) and is therefore inextricably integrated in the package (1), wherein the width (11a) of the elongated slot (11) is larger than an outer diameter (102a) of the syringe tip (102), wherein the syringe tip (102) projects through the elongated slot (11), wherein an abutment of a proximal end face (103b) of the tip cap (103) with an inner surface (10a) of the engagement wall (10) of the first receiving element (2), adjacent to the elongated slot (11), constitutes the engagement of the tip cap (103) with the receiving element (2).

2. System (200) according to claim 1,
wherein the package (1) comprises a package body (3) forming a first cavity (4) defined by a sidewall (5) and a bottom wall (6), wherein the prefilled syringe (100) is arrangeable in said first cavity (4), wherein at least one second receiving element (7, 7a, 7b) is provided in the first cavity (4) of the package body (4), in which the syringe barrel (100) is receivable, wherein at least one third receiving element (8) is provided in the first cavity (4) of the package body (3), in which a rod (104) of the prefilled syringe (100) is receivable, wherein the bottom wall (6) of the package body (3) is provided with a base element (9) above which the first receiving element (7, 7a) is arranged at least in part, wherein the base element (9) and the first receiving element (2) are adjacent to said second receiving element (7, 7a).

3. System (200) according to claims 1 or 2,
wherein the first receiving element (2) comprises a concave shaped body (12) formed by a sidewall (13) and a top wall (14), wherein the engagement wall (10) is part of said sidewall (14) of the concave shaped body (12), wherein the concave shaped body (12) is connected to a base element (9) of the package body (3).

4. System (200) according to claims 1 or 2,
wherein the package (1) comprises a closure element (15), which covers the package body (3) at least in part, wherein the closure element (15) is slidably arranged on the package body (3), wherein the first receiving element (2) is formed by the closure element (15) at least in part and the package body (3) at least in part, wherein the closure element (15) comprises the engagement wall (10), wherein, by a sliding movement of the closure element (15), the engagement of the tip cap (103) with the receiving element (2) is constituted.

5. System (200) according to claim 4,
wherein the closure element (15) is provided with a recess (16), which has essentially the contour of the prefilled syringe (100) with an additional grasp section (20), which allows the user to grasp the prefilled syringe (100), wherein the recess (16) extends along a longitudinal axis (X) of the package body (3) and/or a longitudinal axis (X') of the prefilled syringe (100), wherein the length (17) of the recess (16) is essentially the length (106) of the prefilled syringe (100), wherein the width (19) of a distal section (18) of the recess (16) is essentially the width (108) of the at least one second receiving element (7, 7a, 7b), wherein a grasp section (20) of the recess (16) is suitable and intended for a user to grasp the prefilled syringe (100), wherein a width (22) of a flange section (21) of the recess (16) is essentially the width (107) of the finger flange (105) of the prefilled syringe (100).

6. System (200) according to claim 5,
wherein the closure element (15) is slidable from a closed position to an open position, wherein, in the open position of the closure element (15), the flange section (21) of the recess (16) is positioned along a vertical axis (Z) above the finger flange (105) of the prefilled syringe (100) arranged in the package body (3), as a result of which the prefilled syringe (100) is retrievable from the package (1), wherein, in the closed position, a blocking section (23) of the closure element (15) is positioned along a vertical axis (Z) above the finger flange (105), as a result of which the prefilled syringe (100) is not retrievable from the package (1).

7. System (200) according to claims 1 to 6,
wherein the system (200) comprises a replacement cap (300) for the prefilled syringe (100), wherein the replacement cap (300) comprises an inner cap (301) for an engagement with the syringe tip (102), an outer collar (302) for mounting the replacement cap (300) on the syringe barrel (101) and a grip portion (303) for a user to grasp the replacement cap (300), wherein the replacement cap (300) is arranged in the package (1).

8. System (200) according to claim 7,
wherein the first cavity (4) of the package body (3) is provided with at least one forth receiving element (24), in which the replacement cap (300) is received, wherein an opening (304) of the inner cap (301) is oriented along the longitudinal axis (X) of the package body (3) or along the vertical axis (Z) of the package body (3).

9. System (200) according to claim 7,
wherein the replacement cap (300) is arranged in a second cavity (25) of the package body (3), wherein the second cavity (25) of the package body (3) is adjacent to the first cavity (4) of the package body (3) along the longitudinal axis (X) of the package body (3), wherein an opening (304) of the inner cap (301) is oriented along the vertical axis (Z) of the package body (3).

## Patentansprüche

1. System (200) umfassend eine vorgefüllten Spritze (100) mit einem Spritzenzylinder (101) und einer Spritzenspitze (102) und einer Verpackung (1), die zur Aufnahme der vorgefüllten Spritze (100) geeignet und vorgesehen ist, wobei die Spritzenspitze (102) mit einer Verschlusskappe (103) vorgesehen ist,
**dadurch gekennzeichnet, dass**
die Verschlusskappe (103) zumindest teilweise in ein erstes Aufnahmeelement (2) der Verpackung (1) eingebettet ist, wobei die Verschlusskappe (103) durch einen Eingriff der Verschlusskappe (103) mit dem ersten Aufnahmeelement (2) von der Spritzenspitze (102) abnehmbar ist, wobei das erste Aufnahmeelement (2) eine Eingriffswand (10) mit einem länglichen Schlitz (11) umfasst, der sich entlang einer vertikalen Achse (Z) des Verpackungskörpers (3) erstreckt, wobei die Breite (11a) des länglichen Schlitzes (11) kleiner ist als ein Außendurchmesser (103a) der Verschlusskappe (103), wobei das Aufnahmeelement (2) einen Hohlkörper bildet, wobei die Verschlusskappe (103) nach dem Lösen von der Spritzenspitze (102) in einem Hohlraum des Hohlkörpers verbleibt und somit untrennbar mit der Verpackung (1) verbunden ist, wobei die Breite (11a) des länglichen Schlitzes (11) größer ist als ein Außendurchmesser (102a) der Spritzenspitze (102), wobei die Spritzenspitze (102) durch den länglichen Schlitz (11) hindurchragt, wobei ein Anstoßen einer proximalen Endfläche (103b) der Verschlusskappe (103) an eine Innenfläche (10a) der Eingriffswand (10) des ersten Aufnahmeelements (2), die an den länglichen Schlitz (11) angrenzt, den Eingriff der Verschlusskappe (103) mit dem Aufnahmeelement (2) darstellt.

2. System (200) nach Anspruch 1,
wobei die Verpackung (1) einen Verpackungskörper (3) umfasst, der einen ersten Hohlraum (4) ausbildet, der durch eine Seitenwand (5) und eine Bodenwand (6) definiert ist, wobei die vorgefüllte Spritze (100) in dem Hohlraum (4) des Verpackungskörpers (3) vorgesehen ist, wobei zumindest ein zweites Aufnahmeelement (7. 7a, 7b) in dem ersten Hohlraum des Verpackungskörpers (3) vorgesehen ist, in dem der Spritzenzylinder (101) aufnehmbar ist, wobei zumindest ein drittes Aufnahmeelement (8) in dem ersten Hohlraum (4) des Verpackungskörpers (3) vorgesehen ist, in dem ein Stab (104) der vorgefüllten Spritze (100) aufnehmbar ist, wobei die Bodenwand (6) des Verpackungskörpers (3) mit einem Bodenelement (9) versehen ist, über dem das zweite Aufnahmeelement (7, 7a) zumindest teilweise angeordnet ist, wobei das Bodenelement (9) und das erste Aufnahmeelement (2) an das zweite Aufnahmeelement (7, 7a) angrenzen.

3. System (200) nach einem der Ansprüche 1 oder 2,
wobei das erste Aufnahmeelement (2) einen konkav geformten Körper (12) umfasst, der von einer Seitenwand (13) und einer oberen Wand (14) ausgebildet ist, wobei die Eingriffswand (10) Teil der Seitenwand (14) des konkav geformten Körpers (12) ist, wobei der konkav geformte Körper (12) mit einem Basiselement (9) des Verpackungskörpers (3) verbunden ist.

4. System (200) nach einem der Ansprüche 1 oder 2,
wobei die Verpackung (1) ein Verschlusselement (15) umfasst, das den Verpackungskörper (3) zumindest teilweise abdeckt, wobei das Verschlusselement (15) verschiebbar auf dem Verpackungskörper (3) angeordnet ist, wobei das erste Aufnahmeelement (2) zumindest teilweise durch das Verschlusselement (15) und den Verpackungskörper (3) ausgebildet ist, wobei das Verschlusselement (15) die Eingriffswand (10) umfasst, wobei durch eine Verschiebebewegung des Verschlusselements (15) der Eingriff der Verschlusskappe (103) mit dem Aufnahmeelement (2) gebildet wird

5. System (200) nach Anspruch 4,
wobei das Verschlusselement (15) mit einer Aussparung (16) vorgesehen ist, die im Wesentlichen die Kontur der vorgefüllten Spritze (100) aufweist, mit einem zusätzlichen Griffabschnitt (20), der dem Benutzer das Greifen der vorgefüllten Spritze (100) ermöglicht, wobei sich die Aussparung (16) entlang einer Längsachse (X) des Verpackungskörpers (3) und/oder einer Längsachse (X') der vorgefüllten Spritze (100) erstreckt, wobei die Länge (17) der Aussparung (16) im Wesentlichen die Länge (106) der vorgefüllten Spritze (100) ist, wobei die Breite (19) eines distalen Abschnitts (18) der Aussparung (16) im Wesentlichen die Breite (108) des zumindest einen zweiten Aufnahmeelements (7, 7a, 7b) ist, wobei ein Griffabschnitt (20) der Aussparung (16) zum Greifen der vorgefüllten Spritze (100) durch einen Benutzer geeignet und bestimmt ist, wobei eine Breite (22) eines Flanschabschnitts (21) der Aussparung (16) im Wesentlichen die Breite (107) des Fingerflansches (105) der vorgefüllten Spritze (100) ist.

6. System (200) nach Anspruch 5,
wobei das Verschlusselement (15) aus einer geschlossenen Position in eine offene Position verschiebbar ist, wobei in der offenen Position des Verschlusselements (15) der Flanschabschnitt (21) der Aussparung (16) entlang einer vertikalen Achse (Z) oberhalb des Fingerflansches (105) der im Verpackungskörper (3) angeordneten vorgefüllten Spritze (100) positioniert ist, wodurch die vorgefüllte Spritze (100) aus der Packung (1) entnehmbar ist, wobei in der geschlossenen Position ein Blockabschnitt (23) des Verschlusselements (15) entlang einer vertikalen Achse (Z) oberhalb des Fingerflansches (105) positioniert ist, wodurch die vorgefüllte Spritze (100) nicht aus der Packung (1) entnehmbar ist

7. System (200) nach einem der Ansprüche 1 bis 6,
wobei das System (200) eine Ersatzkappe (300) für die vorgefüllte Spritze (100) umfasst, wobei die Ersatzkappe (300) eine innere Kappe (301) für einen Eingriff mit der Spritzenspitze (102), einen äußeren Kragen (302) zum Befestigen der Ersatzkappe (300) auf dem Spritzenzylinder (101) und einen Griffabschnitt (303) für einen Benutzer zum Ergreifen der Ersatzkappe (300) umfasst, wobei die Ersatzkappe (300) in der Verpackung (1) angebracht ist.

8. System (200) nach Anspruch 7,
wobei der erste Hohlraum (4) des Verpackungskörpers (3) mit zumindest einem vierten Aufnahmeelement (24) vorgesehen ist, in dem die Ersatzkappe (300) aufgenommen ist, wobei eine Öffnung (304) der Innenkappe (301) entlang der Längsachse (X) des Verpackungskörpers (3) oder entlang der vertikalen Achse (Z) des Verpackungskörpers (3) ausgerichtet ist.

9. System (200) nach Anspruch 7,
wobei die Ersatzkappe (300) in einem zweiten Hohlraum (25) des Verpackungskörpers (3) angeordnet ist, wobei der zweite Hohlraum (25) des Verpackungskörpers (3) an den ersten Hohlraum (4) des Verpackungskörpers (3) entlang der Längsachse (X) des Verpackungskörpers (3) angrenzt, wobei eine Öffnung (304) der Innenkappe (301) entlang der vertikalen Achse (Z) des Verpackungskörpers (3) ausgerichtet ist.

## Revendications

1. Système (200) comprenant une seringue préremplie (100) avec un corps de seringue (101) et un embout de seringue (102) et un emballage (1) adapté et destiné à recevoir la seringue préremplie (100), l'embout de seringue (102) étant muni d'un capuchon d'embout (103), **caractérisé par le fait que**
le capuchon d'embout (103) est au moins partiellement intégré dans un premier élément de réception (2) de l'emballage (1), le capuchon d'embout (103) étant détachable de l'embout de seringue (102) par un engagement du capuchon d'embout (103) avec le premier élément de réception (2), le premier élément de réception (2) comprenant une paroi d'engagement (10) avec une fente allongée (11) s'étendant le long d'un axe vertical (Z) du corps d'emballage (3), la largeur (11a) de la fente allongée (11) étant inférieure à un diamètre extérieur (103a) du capuchon d'embout (103), l'élément de réception (2) formant un corps creux, le capuchon d'embout (103) restant dans une cavité du corps creux après avoir été détaché de l'embout de seringue (102) et étant donc inextricablement intégré dans l'emballage (1), la largeur (11a) de la fente allongée (11) étant supérieure à un diamètre extérieur (102a) de l'embout de seringue (102), l'embout de seringue (102) se projetant à travers la fente allongée (11), une butée d'une face d'extrémité proximale (103b) du capuchon d'embout (103) avec une surface intérieure (10a) de la paroi d'engagement (10) du premier élément de réception (2), adjacente à la fente allongée (11), constituant l'engagement du capuchon d'embout (103) avec l'élément de réception (2).

2. Système (200) selon la revendication 1, dans lequel l'emballage (1) comprend un corps d'emballage (3) formant une première cavité (4) définie par une paroi latérale (5) et une paroi inférieure (6), la seringue préremplie (100) étant apte à être placée dans ladite première cavité (4), au moins un deuxième élément de réception (7, 7a, 7b) étant prévu dans la première cavité (4) du corps d'emballage (4), dans lequel le corps de seringue (101) est apte à être reçu, au moins un troisième élément de réception (8) étant prévu dans la première cavité (4) du corps d'emballage (3), dans lequel une tige (104) de la seringue préremplie (100) est apte à être reçue, la paroi inférieure (6) du corps d'emballage (3) comportant un élément de base (9) au-dessus duquel le premier élément de réception (7, 7a) est disposé au moins en partie, l'élément de base (9) et le premier élément de réception (2) étant adjacents audit deuxième élément de réception (7, 7a).

3. Système (200) selon les revendications 1 ou 2, dans lequel le premier élément de réception (2) comprend un corps de forme concave (12) formé par une paroi latérale (13) et une paroi supérieure (14), la paroi d'engagement (10) faisant partie de ladite paroi latérale (14) du corps de forme concave (12), le corps de forme concave (12) étant relié à un élément de base (9) du corps d'emballage (3).

4. Système (200) selon les revendications 1 ou 2, dans lequel l'emballage (1) comprend un élément de fermeture (15), qui recouvre le corps d'emballage (3) au moins en partie, l'élément de fermeture (15) étant disposé de manière coulissante sur le corps d'emballage (3), le premier élément de réception (2) étant formé au moins en partie par l'élément de fermeture (15) et au moins en partie par le corps d'emballage (3), l'élément de fermeture (15) comprenant la paroi d'engagement (10), l'engagement du capuchon d'embout (103) avec l'élément de réception (2) étant constitué par un mouvement de coulissement de l'élément de fermeture (15).

5. Système (200) selon la revendication 4, dans lequel l'élément de fermeture (15) comporte un évidement (16), qui présente essentiellement le contour de la seringue préremplie (100) avec une section de préhension supplémentaire (20), qui permet à l'utilisateur de saisir la seringue préremplie (100), l'évidement (16) s'étendant le long d'un axe longitudinal (X) du corps d'emballage (3) et/ou d'un axe longitudinal (X') de la seringue préremplie (100), la longueur (17) de l'évidement (16) étant essentiellement la longueur (106) de la seringue préremplie (100), la largeur (19) d'une section distale (18) de l'évidement (16) étant essentiellement la largeur (108) de l'au moins un deuxième élément de réception (7, 7a, 7b), une section de préhension (20) de l'évidement (16) étant adaptée et destinée à permettre à un utilisateur de saisir la seringue préremplie (100), une largeur (22) d'une section de bride (21) de l'évidement (16) étant essentiellement la largeur (107) de la bride pour doigt (105) de la seringue préremplie (100).

6. Système (200) selon la revendication 5, dans lequel l'élément de fermeture (15) est apte à coulisser d'une position fermée à une position ouverte, dans la position ouverte de l'élément de fermeture (15) la section de bride (21) de l'évidement (16) étant positionnée le long d'un axe vertical (Z) au-dessus de la bride pour doigt (105) de la seringue préremplie (100) disposée dans le corps d'emballage (3), de telle sorte que la seringue préremplie (100) peut être récupérée de l'emballage (1), dans la position fermée une section de blocage (23) de l'élément de fermeture (15) étant positionnée le long d'un axe vertical (Z) au-dessus de la bride pour doigt (105), de telle sorte que la seringue préremplie (100) ne peut pas être récupérée de l'emballage (1).

7. Système (200) selon les revendications 1 à 6, le système (200) comprenant un capuchon de remplacement (300) pour la seringue préremplie (100), le capuchon de remplacement (300) comprenant un capuchon interne (301) pour un engagement avec l'embout de seringue (102), un collier externe (302) pour monter le capuchon de remplacement (300) sur le corps de seringue (101) et une partie de préhension (303) permettant à un utilisateur de saisir le capuchon de remplacement (300), le capuchon de remplacement (300) étant disposé dans l'emballage (1).

8. Système (200) selon la revendication 7, dans lequel la première cavité (4) du corps d'emballage (3) comporte au moins un quatrième élément de réception (24), dans lequel le capuchon de remplacement (300) est reçu, une ouverture (304) du capuchon interne (301) étant orientée le long de l'axe longitudinal (X) du corps d'emballage (3) ou le long de l'axe vertical (Z) du corps d'emballage (3).

9. Système (200) selon la revendication 7, dans lequel le capuchon de remplacement (300) est disposé dans une seconde cavité (25) du corps d'emballage (3), la seconde cavité (25) du corps d'emballage (3) étant adjacente à la première cavité (4) du corps d'emballage (3) le long de l'axe longitudinal (X) du corps d'emballage (3), une ouverture (304) du capuchon interne (301) étant orientée le long de l'axe vertical (Z) du corps d'emballage (3).
